Europäisches Patentamt

European Patent Office

Office européen des brevets

(11) Veröffentlichungsnummer: **0 253 226**
**B1**

(12) # EUROPÄISCHE PATENTSCHRIFT

(45) Veröffentlichungstag der Patentschrift:
06.06.90

(51) Int. Cl.⁵: **A61K 31/64**, A61K 9/08,
A61K 47/00

(21) Anmeldenummer: 87109562.6

(22) Anmeldetag: 03.07.87

(54) **Spritzfertige, wässrige, alkalische Injektionslösung von Torasemid und Verfahren zu Ihrer Herstellung.**

(30) Priorität: 12.07.86 DE 3623620

(43) Veröffentlichungstag der Anmeldung:
20.01.88 Patentblatt 88/3

(45) Bekanntmachung des Hinweises auf die Patenterteilung:
06.06.90 Patentblatt 90/23

(84) Benannte Vertragsstaaten:
AT BE CH DE ES FR GB GR IT LI LU NL SE

(56) Entgegenhaltungen:
EP-A- 0 030 681
EP-A- 0 185 374
DE-A- 3 241 765
FR-A- 2 261 011

ARZNEIMITTEL FORSCHUNG/DRUG RESEARCH,
Band 35 (II), Oktober 1985, Seiten 1532-1535, Edito
Cantor, Aulendorf, DE; H. HERMES et al.: "Renal effects
of torasemide in the rat"
REMINGTON'S PHARMACEUTICAL SCIENCES, 16.
Auflage, 1980, Seiten 1466-1468, Mack Publishing Co.,
Easton, US; Seite 1466, rechte Spalte, Zeile 65 -
Seite 1468, Zeile 9

(73) Patentinhaber: BOEHRINGER MANNHEIM GMBH,
Sandhofer Strasse 116, D-6800 Mannheim 31(DE)

(72) Erfinder: Demmer, Fritz, Dr. rer. nat., Kastanienweg 21,
D-6945 Hirschberg-Leutershausen(DE)
Erfinder: Gruber, Werner, Am Heiligenberg 12,
D-6943 Birkenau(DE)
Erfinder: Woog, Heinrich, Dr. rer. nat., Lindenstrasse 6,
D-6941 Laudenbach(DE)

**Beschreibung**

Gegenstand der vorliegenden Erfindung sind neue spritzfertige, wässrige, alkalische Injektionslösungen von Torasemid, welche stabil und venenverträglich sind, sowie Verfahren zu ihrer Herstellung.

Aus der DE–A 25 160 252 sind Pyridinderivate mit entzündungshemmenden und diuresefördernden Eigenschaften bekannt. Gemäß dieser Literaturstelle sollen diese Verbindungen als Dragees, Tabletten, Kapseln oder Stuhlzäpfchen in Dosierungen von 50 bis 300mg Aktivsubstanz verabreicht werden. Als interessanteste Verbindung dieser Gruppe wurde in der Zwischenzeit 1-Isopropyl-3-((4m-toluidino-3-pyridyl)sulfonyl)-Harnstoff erkannt, der hervorragende diuretische Eigenschaften besitzt. Die Substanz hat inzwischen den generischen Namen «Torasemid» erhalten.

Die pharmakologische Wirkung von Torasemid ist ausführlich in Arzneimittel Forschung/Drug Research, (1985), 35 (II), 1532–35 beschrieben. Dort wurde insbesondere die renale Wirkung von Torasemid auf die glomeruläre Filtrationsrate und die tubuläre Resorption unter Anwendung von Clearance– und Mikropunktionstechniken an Ratten untersucht. Dabei hat sich gezeigt, daß sich Torasemid hinsichtlich der verschiedenen renalen Wirkungen nicht von anderen typischen Schleifendiuretica unterscheidet. Die im Zusammenhang mit den dort beschriebenen Versuchen hergestellten Lösungen von Torasemid hatten einen pH-Wert von 8,5 und erhielten den Wirkstoff in einer Konzentration von 4mg/ml in wässrigem Medium ohne Zusatz eines organischen Lösungsmittels. Diese Lösungen haben jedoch eine begrenzte Lagerfähigkeit von nur ca. 1 Woche, und müssen demzufolge immer wieder frisch hergestellt werden. Als Injektionslösungen, die gegebnenfalls auch über einen längeren Zeitraum aufbewahrt werden müssen, sind diese Lösungen untauglich.

Obwohl eine Dauermedikation mit solchen Substanzen üblicherweise in Form der festen Präparate erfolgt, ist es wünschenswert, insbesondere für die klinische Anwendung, auch eine injizierbare Zubereitungsform zur Verfügung zu haben. Um nicht bei der Anwendung noch darauf angewiesen zu sein, die Komponenten miteinander zu mischen, sollte eine solche Lösung «spritzfertig» sein, d.h. alle notwendigen Komponenten in den richtigen Mischungsverhältnissen bereits in einer Ampulle vereinigt enthalten. Versuche, Torasemid in üblichen injizierbaren Zubereitungen herzustellen, schlugen überraschenderweise fehl. Solche Zubereitungen sind entweder nicht venenverträglich oder nicht genügend stabil, so daß insbesondere bei längerer Lagerung die Lösungen rekristallisieren oder Zersetzungsprodukte ausscheiden, welche eine Verwendung für Injektionszwecke ausschließen.

Es stellt sich daher die Aufgabe, eine Rezeptur zu finden, mit der Torasemid in der benötigten Konzentration von 2 bis 40 mg/ml in eine verträgliche, lagerstabile injizierbare Lösung gebracht werden kann.

Diese Aufgabe wird gelöst, in dem man spritzfertige, wässrige, alkalische Injektionslösungen von Torasemid zur Verfügung stellt, die einen physiologisch verträglichen alkalischen Puffer mit einem ph-Wert von 9,3–9,9 und einer Pufferkapazität von bis zu 0,1 val/l und ein organisches Lösungsmittel in einer Menge von 5–20 Gew.% enthält, wobei das organische Lösungsmittel aus der Gruppe der Polyethylenglykole mit einem Molgewicht von 100–1500, Polypropylenglykole mit einem Molgewicht von 50–1000, Glycerin, Proplenglykol, Ethanol und Propanol ausgewählt ist und das Torasemid in einer Konzentration von 2–40 mg/ml enthalten ist.

Die Löslichkeit von Torasemid als Natrium- oder Kaliumsalz beträgt in Wasser etwa 250mg/ml, wobei sich ein ph-Wert von ungefähr 9,5 einstellt, da Torasemid eine relativ schwache Säure ist. Diese Löslichkeit sollte an sich vollständig ausreichen, um Injektionslösungen, die nur etwa 2–40 mg/ml Torasemid enthalten müssen, herzustellen.

Wie vorstehend gesagt, scheiden jedoch solche Lösungen beim Stehenlassen Kristalle aus, so daß sie als spritzfertige Injektionslösungen ungeeignet sind. Eine Erhöhung des ph-Wertes der Lösung auf einen Wert von über 10 verhindert zwar ein Auskristallisieren des Torasemids, führt jedoch zu einer Venenunverträglichkeit der Lösung. Darüber hinaus bilden sich nach einiger Zeit schwer lösliche Zersetzungsprodukte, die wiederum ausfallen und die vor der Applikation der Lösungen entfernt werden müssen. Darüber hinaus verringert sich in der Lösung langsam der Gehalt an aktivem Wirkstoff. Die Lösung wird somit insgesamt für Injektionszwecke unbrauchbar.

Überraschenderweise erhält man jedoch stabile, partikelfreie, venenverträgliche Injektionslösungen, wenn man der Lösung einerseits einen physiologisch verträglichen, alkalischen Puffer mit einem ph-Wert zwischen 9,3 und 9,9 und andererseits ein organisches Lösungsmittel aus der Gruppe Polyethylenglycol, Polypropylenglycol, Glycerin, Propylenglykol, Äthanol und Propanol zusetzt. Eine ausreichende Venenverträglichkeit ist gewährleistet, wenn die Pufferkapazität unter 0,1 val/l und die Menge des Lösungsmittels in einem Bereich von 5–20 Gew.% gehalten wird. Wirkstoffmenge und Zusätze sind so einzurichten, daß nach Möglichkeit isotonische Lösungen erhalten werden. Soweit dies durch die vorbeschriebenen Komponenten nicht bereits gewährleistet ist, können darüber hinaus auch andere für diesen Zweck gebräuchliche Stoffe, wie Natriumchlorid, Fructose, Glucose etc. zur Einstellung der Isotonie zugesetzt werden.

Als Lösungsmittel sind Polyethylenglycole mit einem Molgewicht zwischen 100 und 1500, insbesondere einem Molgewicht von 200–600 besonders bevorzugt. Die übrigen vorerwähnten Stoffe können jedoch auch eingesetzt werden, obwohl bei diesen nach längerer Lagerung eine geringfügige Partikelbildung beobachtet wird. Das Lösungsmittel sollte üblicherweise in einer Menge von etwa 5–20 Gew.-% zugesetzt werden, wobei ein Zusatz von etwa 10% bevorzugt ist.

Es hat sich weiterhin als erfindungswesentlich herausgestellt, die verwendeten Lösungsmittel vor ihrem Einsatz weitgehend von Aldehyden und Ketonen zu befreien, wobei ein Aldehydgehalt von unter 30 ppm, vorzugsweise unter 10 ppm eingehalten werden sollte. Bei der Verwendung von sonst üblichen Lösungsmitteln mit einem nicht näher definierten Aldehydgehalt hat sich gezeigt, daß Torasemid bei längerer Lagerung in Lösung teilweise in verschiedene Verbindungen zerfällt und daß diese Zersetezungsprodukte wiederum mit den in der Lösung vorhandenen Aldehyden oder Ketonen zu besonders schwer löslichen Verbindungen reagieren, die als kleine Partikel aus der Lösung ausfallen. Um diesem Prozess vorzubeugen, kommen vorzugweise solche Lösungsmittel zur Anwendung, deren Aldehydanteil innerhalb der oben genannten Grenzen liegt. Die injizierbare Lösung von Torasemid sollte maximal einen Gesamtaldehydgehalt von 30 ppm aufweisen. Der Begriff "Aldehyd" steht sowohl für Formaldehyd als auch für die höheren homologen Aldehyde, die sich durch Oxidation bzw. Zerfall der jeweils als organische Lösungsmittel verwendeten Alkohole bilden können.

Üblicherweise erfolgt eine Reinigung der in Frage kommenden Lösungsmittel durch eine entsprechende Destillation, jedoch ist natürlich auch eine Zugabe von geeigneten Reduktionsmitteln, beispielsweise Metallhydriden oder Metallen, wie Lithium, Natrium oder Kalium möglich, zumal die damit gebildeten Alkalimetallhydroxide anschließend zur Lösung des Torasemids dienen können. Um eine nachträgliche Bildung von solchen Aldehyden oder Ketonen zu vermeiden, werden die Injektionslösungen in sauerstofffreier Atmosphäre, beispielsweise unter Stickstoff, hergestellt und gelagert. Auch der Zusatz kleiner Mengen physiologisch verträglicher Reduktionsmittel, beispielsweise Ascorbinsäure, kann zu diesem Zweck sinnvoll sein.

Als physiologisch verträgliche Puffer kommen insbesondere die Natrium-, Kalium- oder Ammoniumsalz schwacher Säuren, z.B. Carbonat, Phosphat, Glycinat, Arginat, N-Methylglucosaminat oder andere Aminosäuren in Frage, jedoch kann auch z. B. das gut verträgliche Tris-(hydroxymethyl)-aminomethan (Trometamol) mit Vorteil eingesetzt werden. Ein Spezialfall der Pufferung kann auch in dem Zusatz eines puffernd wirkenden weiteren Wirkstoffes, beispielsweise in dem Zusatz von Canrenoat oder Furosemid-Natrium oder Kaliumsalzen bestehen. Da das Torasemid in der Injektionslösung in einer Menge von etwa 0,01 bis 0,2 Mol/1 enthalten ist, hat sich eine Puffermenge von 0,01 bis 0,1 val/l als besonders günstig erwiesen. Dieser Bereich kombiniert eine ausreichende Pufferung mit einer guten Venenverträglichkeit, die bei höheren Pufferkonzentrationen insbesondere nicht mehr gegeben ist.

Die Herstellung der erfindungsgemäßen Injektionslösungen erfolgt vorzugsweise so, daß man den Wirkstoff in dem organischen Lösungsmittel mit einem Teil des benötigten Wassers suspendiert und durch Zugabe von wässrigem Alkali oder Ammoniak in Lösung bringt. Danach werden die Puffer und übrigen Hilfsstoffe zugefügt und durch Zugabe von geringen Mengen Säuren oder Alkali der gewünschte pH-Wert von 9,3 bis 9,9 eingestellt.

Selbstverständlich kann alternativ auch direkt das Torasemidsalz selbst eingesetzt werden.

Weiterhin hat sich gezeigt, daß es vorteilhaft ist, wenn zur Herstellung der Injektionslösung das Torasemid mit einer Partikelgröße von unter 10 /um, insbesondere unter 2 /um eingesetzt wird.

Die so erhaltene Lösung wird von ggf. vorhandenen Partikeln abfiltriert und sterilisiert. Für die Sterilisation hat sich insbesondere ein Erhitzen auf 100 bis 130°C bewährt, da dies weniger aufwendig ist als eine Sterilfiltration. Vor der Hitzesterilisation wird üblicherweise bereits auf Ampullen abgefüllt, wobei Ampullengrößen von 5 bis 20 ml mit Wirkstoffgehalten von 10 bis 200 mg Wirkstoff pro Ampulle sich bewährt haben. Die Ampullen werden üblicherweise unter Stickstoff abgefüllt und erweisen sich bei Raumtemperatur als mindestens drei Jahre lang lagerfähig, ohne daß Trübungen auftreten oder der Wirkstoff in signifikantem Maße chemisch verändert wird.

Wegen der geringen Pufferkapazität führen die Lösungen nicht zu signifikanten pH-Wert-Veränderungen an der Injektionsstelle, so daß eine unverdünnte Applikation möglich ist. Die erfindungsgemäßen Lösungen können jedoch auch mit einer isotonischen Glucose oder Kochsalzlösung gemischt und infundiert werden.

Zur Herstellung von isotonischen und/oder injizierbaren Lösungen ist es natürlich auch möglich, zunächst solche Lösungen herzustellen, die eine höhere Pufferkapazität als 0.1 val/l aufweisen, und diese dann durch Zugabe einer entsprechenden Säure, beispielsweise Salzsäure, auf einen pH-Wert von 7,0 - 7,5, vorzugsweise jedoch auf den pH-Wert des Blutes von 7,4 zu bringen, wobei gleichzeitig durch die vorgenommene Verdünnung der Lösung die isotonen spritzfertigen Injektionslösungen erhalten werden.

Die erfindungsgemäßen Injektionslösungen können selbstverständlich auch noch die üblichen Hilfs- oder Zusatzstofe enthalten, die sie für die Applikation geeignet nachen, beispielsweise Tenside, Mineralstoffe, Vitamine etc.

In den nachfolgenden Beispielen wird die Erfindung näher erläutert, ohne sie dadurch begrenzen zu wollen.

Beispiel 1:

Torasemid 20 mg/4 ml Injektionslösung

In einem sterilen mit Rührwerk versehenen 50 l V2A-Doppelmantelkessel werden 30 l Wasser für Injektionszweck und 4,5 kg Polyethylenglykol 400 vorgelegt und unter Stickstoffbegasung, Lichtschutz und Rühren 16,0 g Natriumhydroxid gelöst. Darin werden unter kräftigem Rühren 200 g mikronisiertes Torasemid einsuspendiert und zum Teil gelöst. Mit 8 g Natronlauge in 200 ml Wasser für Injektionszwecke wird das restliche Torasemid gelöst und der pH-WErt auf 9,6 - 9,8 eingestellt, 5 g Tris-(hydroxymethyl)-aminomethan zugesetzt und unter Rühren gelöst. Der Ansatz wird mit Wasser für Injektionszwecke zum Endvolumen von 40 l aufgefüllt und umgerührt. Die Lösung wird über Membranfilter 0,2 µm Porenweite sterilfiltriert. Die sterilfiltrierte Lösung wird zu 4,2 ml in 5 ml Ampullen abgefüllt. Die Ampullen werden bei 121° C 20 min im Autoklaven sterilisiert. Man erhält so eine klare spritzfertige Injektionslösung, die mindestens 3 Jahre ohne Trübung und Zersetzung haltbar ist. Der pH-Wert dieser Lösung beträgt, 9,5, die Tritrationsbasizität entspricht 9,7 ml 0,1 normaler Salzsäure, die pro Ampulle zum Absenken des pH-Wertes von 9,5 auf 7,4 benötigt wird.

Beispiel 2:

Torasemid 200 mg/20 ml Injektionslösung in Form von Torasemid-Natrium

In einem sterilen mit Rührwerk versehenen 200 l V2A-Doppelmantelkessel werden 150 l Wasser für Injektionszwecke und 22,5 kg Polyethylenglykol 400 vorgelegt und unter Stickstoffbegasung, Lichtschutz und Rühren 198 g Natriumhydroxid gelöst. Darin werden unter kräftigem Rühren 2 kg mikronisiertes Torasemid einsuspendiert und zum Teil gelöst. Mit 40 g Natirumhydroxid in 1000 ml Wasser für Injektionszwecke wird das restliche Torasemid gelöst und der pH-Wert auf 9,6 - 9,8 eingestellt. 15 g Trinatriumphosphat werden zugesetzt und unter Rühren gelöst. Der Ansatz wird mit Wasser für Injektionszwecke zum Endvolumen von 200 l aufgefüllt, umgerührt und über ein Membranfilter mit der Porenweite 0,2 µm sterilfiltriert. Die sterilfiltrierte Lösung wird zu 20,5 ml in Ampullen abgefüllt und bei 121° C 20 min im Autoklaven sterilisiert.
Man erhält so eine klare, spritzfertige Injektionslösung, die mindestens 3 Jahre ohne Trübung und Zerstezung haltbar ist. Der pH-Wert der Injektionslösung beträgt 9,5. Die Titrationsbasizität entspricht 19,4 ml 0,1 normaler Salzsäure, die pro Ampulle zum Absenken des pH-Wertes von 9,5 auf 7,4 benötigt wird.

Beispiel 3:

Torasemid 100 mg/10 ml Injektionslösung in Form von Torasemid-Kalium

In einem sterilen mit Rührwerk versehenen 100 l V1A-Doppelmantelkessel werden 75 l Wasser für Injektionszwecke und 10 l Äthanol vorgelegt und unter Stickstoffbegasung, Lichtschutz und Rühren 150 g Kaliumhydroxid gelöst. Darin werden unter kräftigem Rühren 1 kg mikronisiertes Torasemid einsuspendiert und zum Teil gelöst. Mit 62 g Kaliumhydroxid in 500 ml Waser für Injektionszwecke wird das restliche Torasemid gelöst und der pH-Wert auf 9,6 - 9,8 eingestellt. 5 g Kaliumcrbonat werden zugesetzt und unter Rühren gelöst. Der Ansatz wird mit Wasser für Injektionszwecke zum Endvolumen von 100 l aufgefüllt, umgerührt und über ein Membranfilter mit einer Porenweite von 0,2 µm sterilfiltriert. Die sterilfiltrierte Lösung wird zu 10,5 ml pro Ampulle abgefüllt und bei 121° C 20 min im Autoklaven sterilisiert. Man erhält so eine klare, spritzfertige Injektionslösung, die mindestens 3 Jahre ohne Trübung und Zersetzung haltbar ist. Der pH-Wert der Injektionslösung beträgt 9,5, die Titrationsbasizität entspricht 9,8 ml 0,1 normaler Salzsäure,die pro Ampulle zum Absenken des pH-Wertes von 9,5 auf 7,4 benötigt wird.

Beispiel 4:

Torasemid 100 mg/10 ml Injektionslösung in Form von Torasemid-kalium und Arginin als Puffer

In einem sterilen mit Rührwerk versehenen 100 l V2A-Doppelmantelkessel werden 75 l Wasser für Injektionszwecke und 10 l Ethanol vorgelegt und unter Stickstoffbegasung, Lichtschutz und Rühren 150 g Kaliumhydroxid gelöst. Darin werden unter kräftigem Rühren 1 kg mikronisiertes Torasemid einsuspendiert und zum Teil gelöst. Mit 62 g Kaliumhydroxid in 500 ml Wasser für Injektionszwecke wird das restliche Torasemid gelöst und der pH-Wert auf 9,6 - 9,8 eingestellt. 5 g Arginin werden zugesetzt und unter Rühren gelöst. Der Ansatz wird umgerührt und über ein Membranfilter mit einer Porenweite von 0,2 µm filtriert. Die filtrierte Lösung wird zu 10,5 ml pro Ampullen abgefüllt und bei 121° C 20 min im Autoklaven sterilisiert. Man erhält so eine klare, spritzfertige Injektionslösung, die mindestens 3 Jahre ohne Trübung und Zersetzung haltbar ist. Der pH-Wert der Injektionslösung beträgt 9,5 die Titrationsbasizität entspricht 9,8 ml 0,1 normaler Salzsäure, die pro Ampulle zum Absenken des pH-Wertes von 9,5 auf 7,4 benötigt wird.

Beispiel 5:

Vergleichende Partikelmessungen bei Torasemid-Injektionslösungen

| Rezeptur Zusammensetzung | 86/1 | 86/2 | 86/3 | 86/4 | 86/5 |
|---|---|---|---|---|---|
| Torasemid | 200,0 mg | 200,0 mg | 200,0 mg | 200,0 mg | 200,0 mg |
| Trometamol | 1,0 mg | 1,0 mg | 1,0 mg | 1,0 mg | 1,0 mg |
| NaOH | 23,8 mg | 23,8 mg | 23,8 mg | 23,8 mg | 23,8 mg |
| PEG 400 | 2,0 ml | – | – | – | 1,0 ml |
| Ethanol | – | 2,0 ml | – | – | 1,0 ml |
| Propylenglykol | – | – | 2,0 ml | – | – |
| Wasser für Injekt. ad | 20,0 ml | 20,0 ml | 20,0 ml | 20,0 ml | 20,0 ml |

Die Partikelzahl wird gemäß britischer Pharmakopoe II, 1980, Seite 578 bzw. US Pharmakopoe XXI, 1985, 1258 bestimmt. Gemessen wurden Partikel mit Durchmessern größer 2 μm und Partikel größer 25 μm, die in der folgenden Tabelle jeweils übereinander angeführt sind.

Partikelmessungen:

| Versuchsnr. | nach 24 h | nach 1 Wo. | nach 4 Wo. | nach 8 Wo. |
|---|---|---|---|---|
| 86/1 | 164 | 108 | 134 | 67 |
| | 0 | 0 | 0 | 0 |
| 86/2 | 234 | 287 | 647 | 626 |
| | 1 | 1 | 1 | 10 |
| 86/3 | 310 | 470 | 682 | 658 |
| | 2 | 4 | 4 | 43 |
| 86/4 | 444 | 898 | 1297 | 2460 |
| | 3 | 2 | 23 | 35 |
| 86/5 | 181 | 203 | 390 | 420 |
| | 0 | 1 | 3 | 7 |

Beispiel 6:

Kombinationspräparat mit 200 mg Furosemid und 10 mg Torasemid/10 ml Injektionslösung

In 6 l Wasser für Injektionszwecke werden 144,48 g Furosemid suspendiert und durch Zugabe von 5%iger Kalilauge gelöst. Danach suspendiert man 8 g Torasemid in diese Lösung und stellt mit 5-%iger Kalilauge den pH-Wert auf 11,0 ein. Nun gibt man 18,4 g Natriumcarbonat, 0.8 l Polyethylenglykol 400 (PEG. 400) sowie ad 8 l Wasser für Injektionszwecke dazu, rührt kräftig um und stellt den pH-Wert nochmals mit Kalilauge auf 11,0 ein. Die Lösung wird über Membranfilter mit 0,2 μm Porenweite sterilfiltriert. Die sterilfiltrierte Lösung wird zu 10,2 ml in 10 ml Ampullen abgefüllt. Die Ampullen werden bei 121° C 20 min im Autoklaven sterilisiert. Man erhält so eine klare, spritzfertige Injektionslösung, die mindestens 3 Jahre ohne Trübung und Zersetzung haltbar ist. Der pH-Wert dieser Lösung liegt zwischen 10,8 und 11,2. Die Titrationsbasizität entspricht 5,8 ml 0,1 n Salzsäure, die pro Ampulle zum Absenken des pH-Wertes von 11,0 auf 7,4 benötigt werden.

Beispiel 7:

Kombinationspräparat mit 200 mg Kaliumcanrenoat und 10 mg Torasemid/10 ml Injektionslösung

In 6 l Wasser für Injektionszwecke werden 144,48 g Canrenoinsäure suspendiert und durch Zugabe von 5 %iger Kalilauge gelöst. Danach suspendiert man 8 g Torasemid in diese Lösung und stellt mit 5-%iger Kalilauge den pH-Wert auf 11,0 ein. Nun gibt man 18,4 g Natriumcarbonat, 0,8 l Polyethylenglykol

400 sowie ad 8 l Wasser für Injektionszwecke dazu, rührt kräftig um und stellt den pH-Wert nochmals mit Kalilauge auf 11,0 ein. Die Lösung wird über Membranfilter mit 0,2 μm Porenweite sterilfiltriert. Die sterilfiltrierte Lösung wird zu 10,2 ml in 10 ml Ampullen abgefüllt. Die Ampullen werden bei 121° C 20 min im Autoklaven sterilisiert. Man erhält so eine klare, spritzfertige Injektionslösung, die mindestens 3 Jahre ohne Trübung und Zersetzung haltbar ist. Der pH-Wert dieser Lösung liegt zwischen 10,8 und 11,2. Die Titrationsbasizität entspricht 5,8 ml 0,1 n Salzsäure, die pro Ampulle zum Absenken des pH-Wertes von 11,0 auf 7,4 benötigt werden.

<u>Beispiel 8:</u>

<u>Torasemid 10 mg/2 ml Injektionslösung</u>

In einem sterilen, mit Rührwerk versehenen 100 l V2A-Doppelmantelkessel werden 50 l Wasser für Injektionszwecke, 2 kg Polyethylenglykol 400, 1 kg Ethanol und 1,5 kg Propylenglykol vorgelegt und unter Stickstoffbegasung, Lichtschutz und Rühren 16,0 g Natriumhydroxid gelöst. Darin werden unter kräftigem Rühren 200 g mikronisiertes Torasemid einsuspendiert und zum Teil gelöst. Mit 8 g Natronlauge in 200 ml Wasser für Injektionszwecke wird das restliche Torasemid gelöst und der pH-Wert auf 9,6 bis 9,8 eingestellt. 5 g Tris-(hydroxymethyl)-aminomethan werden zugesetzt und unter Rühren gelöst. Der Ansatz wird mit Wasser für Injektionszwecke zum Endvolumen von 80 l aufgefüllt und umgerührt. Die Lösung wird über Membranfilter mit 0,2 μm Porenweite sterilfiltriert. Die sterilfiltrierte Lösung wird zu 2,2 ml in 2 ml Ampullen abgefüllt. Die Ampullen werden bei 121° C 20 min im Autoklaven sterilisiert. Man erhält so eine klare, spritzfertige Injektionslösung, die mindestens 3 Jahre ohne Trübung und Zersetzung haltbar ist. Der pH-Wert dieser Lösung beträgt 9,5, die Titrationsbasizität entspricht 4,8 ml 0,1 n Salzsäure, die pro Ampulle zum Absenken des pH-Wertes von 9,5 auf 7,4 benötigt werden.

**Patentansprüche**

1. Spritzfertige, wässrige, alkalische Injektionslösungen enthaltend Torasemid in einer Konzentration von 2–40 mg/ml, dadurch gekennzeichnet, daß sie einen physiologisch verträglichen alkalischen Puffer mit einem pH-Wert von 9,3–9,9 und einer Pufferkapazität von bis zu 0,1 val/l und ein organisches Lösungsmittel in einer Menge von 5–20 Gew.-%, ausgewählt aus der Gruppe Polyethylenglykole mit einem Molgewicht von 100–1500, Polypropylenglykole mit einem Molgewicht von 50–1000, Glycerin, Propylenglykol, Ethanol und Propanol enthält.

2. Injektionslösung gemäß Anspruch 1, dadurch gekennzeichnet, daß das organische Lösungsmittel weniger als 30 ppm, vorzugsweise weniger als 10 ppm Aldehyde enthält.

3. Injektionslösung gemäß Anspruch 1 oder 2, dadurch gekennzeichnet, daß der Puffer Natrium, Kalium oder Ammoniumcarbonat, -phosphat, Glycinat- oder Arginat-, N-Methylglucosaminat oder ein anderer Aminosäurepuffer und/oder Trishydroxymethylaminomethan ist.

4. Injektionslösung gemäß Anspruch 1 bis 3, dadurch gekennzeichnet, daß zusätzlich ein physiologisch verträgliches Tensid oder ein Reduktionsmittel enthalten ist.

5. Injektionslösung gemäß Anspruch 1 bis 4, dadurch gekennzeichnet, daß ferner Mittel zur Herstellung einer isotonischen Konzentration enthalten sind.

6. Injektionslösung gemäß Anspruch 1 bis 5, dadurch gekennzeichnet, daß ein weiterer pharmakologischer Wirkstoff, insbesondere ein Diuretikum und besonders bevorzugt Canrenoat und/oder Furosemid in einer Menge bis zu 100 mg/ml enthalten ist.

7. Injektionslösung gemäß Anspruch 5, dadurch gekennzeichnet, daß als Mittel zur Einstellung einer isotonischen Konzentration Natriumchlorid, Fructose, Lactose oder Glucose enthalten sind.

8. Injektionslösung gemäß Anspruch 1 bis 7, dadurch gekennzeichnet, daß als organisches Lösungsmittel Polyethylenglykole mit einem Molgewicht von 200–600, insbesondere von 400 eingesetzt werden.

9. Verfahren zur Herstellung von Injektionslösungen gemäß Anspruch 1 bis 6, dadurch gekennzeichnet, daß man das Torasemid in einem organischen Lösungsmittel, ausgewählt aus der Gruppe Polyethylenglykole mit einem Molgewicht von 100–1500, Polypropylenglykole mit einem Molgewicht von 50–1000, Glycerin, Propylenglykol, Ethanol und Propanol, suspendiert, durch Zugabe von wässrigem Alkali in Lösung bringt, den Puffer und die übrigen Hilfsstoffe zufügt, den pH-Wert durch Zugabe von Säuren oder Alkali auf 9,3–9,9 einstellt, von ungelösten Partikeln filtriert und sterilisiert.

10. Verfahren nach Anspruch 9, dadurch gekennzeichnet, daß durch Erhitzen auf 100–130°C sterilisiert wird.

11. Verfahren nach Anspruch 9 oder 10, dadurch gekennzeichnet, daß die Lösung auf Ampullen abgefüllt und unter Stickstoff aufbewahrt wird.

12. Verfahren gemäß Anspruch 9 bis 11, dadurch gekennzeichnet, daß das Torasemid mit einer Partikelgröße von unter 10 μm, vorzugsweise unter 2 μm eingesetzt wird.

13. Verfahren gemäß Anspruch 9, dadurch gekennzeichnet, daß das organische Lösungsmittel weniger als 30 ppm, vorzugsweise weniger als 10 ppm Aldehyde enthält.

**Claims**

1. Aqueous, alkaline injection solution ready for injection containing torasemide in a concentration of 2–40 mg./ml.,characterised in that it contains a physiologically compatible alkaline buffer with a pH value of 9.3–9.9 and a buffer capacity of up to 0.1 val/l and an organic solvent in an amount of 5–20 wt.% selected from the group polyethylene glycols with a molecular weight of 100–1500, polypropylene glycols with a molecular weight of 50–1000, glycerol, propylene glycol, ethanol and propanol.

2. Injection solution according to claim 1, characterised in that the organic solvent contains less than 30 ppm, preferably less than 10 ppm aldehydes.

3. Injection solution according to claim 1 or 2, characterised in that the buffer is sodium, potassium or ammonium carbonate, phosphate, glycinate or arginate, N-methylglucosaminate or another amino acid buffer and/or tris-(hydroxymethyl)-aminomethane.

4. Injection solution according to claim 1 to 3, characterised in that it additionally contains a physiologically compatible tenside or a reducing agent.

5. Injection solution according to claim 1 to 4, characterised in that there are also contained agents for the production of an isotonic solution.

6. Injection solution according to claim 1 to 5, characterised in that there is contained a further pharmacologically active material, especially a diuretic and especially preferably canrenoate and/or furosemide in an amount of up to 100 mg./ml.

7. Injection solution according to claim 5, characterised in that as agents for the adjustment of an isotonic concentration there are contained sodium chloride, fructose, lactose or glucose.

8. Injection solution according to claim 1 to 7, characterised in that, as organic solvents, there are used polyethylene glycols with a molecular weight of 200–600, especially of 400.

9. Process for the preparation of injection solutions according to claim 1 to 6, characterised in that one suspends torasemide in an organic solvent selected from the group polyethylene glycols with a molecular weight of 100–1500, polypropylene glycols with a molecular weight of 50–1000, glycerol, propylene glycol and propanol, brought into solution by the addition of aqueous alkali, the buffer and other adjuvants added thereto, the pH value is adjusted to 9.3 to 9.9 by addition of acid or alkali, filtered from undissolved particles and sterilised.

10. Process according to claim 9, characterised in that it is sterilised by heating to 100–130°C.

11. Process according to claim 9 or 10, characterised in that the solution is filled into ampoules and stored under nitrogen.

12. Process according to claim 9 to 11, characterised in that torasemide is used with a particle size of below 10 μm., preferably below 2 μm.

13. Process according to claim 9, characterised in that the organic solvent contains less than 30 ppm, preferably less than 100 ppm of aldehyde.

**Revendications**

1. Solution injectable alcaline aqueuse, prète à l'injection, contenant du torasemide en une concentration de 2–40 mg/ml, caractérisée en ce qu'elle contient un tampon alcalin physiologiquement acceptable, ayant un pH de 9,3–9,9 et un pouvoir tampon allant jusqu'à 0,1 val/l et un solvant organique en une proportion de 5–20% en poids, choisi parmi les polyéthylèneglycols ayant une masse molaire de 100–1500, les polypropylèneglycols ayant une masse molaire de 50–1000, la glycerine, le propylèneglycol, l'éthanol et le propanol.

2. Solution injectable selon la revendication 1, caractérisée en ce que le solvant organique contient moins de 30 ppm, de préférence moins de 10 ppm d'aldéhyde.

3. Solution injectable selon la revendication 1 ou 2, caractérisée en ce que le tampon est le carbonate, le phosphate, le glycinate, l'arginate, la N-méthylglucosaminate de sodium, de potassium ou d'ammonium, ou un autre tampon à base d'acide amine et/ou le trishydroxymethylaminomèthane.

4. Solution injectable selon les revenidications 1 à 3, caractérisée en ce qu'elle contient en outre un tensioactif ou un agent réducteur physiologiquement acceptable.

5. Solution injectable selon les revendications 1 à 4, caractérisée en ce qu'elle contient en outre un agent permettant l'obtention d'une concentration isotonique.

6. Solution injectable selon les revendications 1 à 5, caractérisée en ce qu'elle contient en outre un autre composant actif pharmacologique, en particulier un diurétique et de façon particulièrement préférée, du canrenoate et/ou du furosemide, en une proportion allant jusqu'à 100 mg/ml.

7. Solution injectable selon la revendication 5, caractérisée en ce que comme agent pour l'obtention d'une concentration isotonique, elle contient du chlorure de sodium, du fructose, du lactose ou du glucose.

8. Solution injectable selon les revendications 1 à 7, caractérisée en ce que comme solvant organique, on utilise des polyéthylèneglycols ayant une masse molaire de 200–600, de préférence de 400.

9. Procédé pour la préparation de solutions injectable selon les revendications 1 à 6, caractérisé en ce que l'on met en suspension le torasemide dans un solvant organique choisi parmi les polyéthylèneglycols ayant une masse molaire de 100–1500, les polypropylèneglycols ayant une masse molaire de 50–

1000, la glycerine, le propylèneglycol, l'éthanol et le propanol, que l'on le solubilise par addition d'alcali aqueux, ajoute le tampon et les autres adjuvants, ajuste le pH par addition d'acide ou d'alcali a une valeur de 9,3–9,9, filtre les particules insolubles et stérilise.

10. Procédé selon la revendication 9, caractérisé en ce que l'on stérilise par chauffage à 100–130°C.

11. Procédé selon la revendication 9 ou 10, caractérisé en ce que l'on met la solution dans des ampoules et on la conserve sous azote.

12. Procédé selon les revendications 9 à 11, caractérisé en ce que l'on utilise un torasemide ayant une taille des particules inférieure à 10 µm, de préférence inférieure à 2 µm.

13. Procédé selon la revendication 9, caractérisé en ce que le solvant organique contient moins de 30 ppm, de préférence moins de 10 ppm d'aldéhyde.